# EUROPEAN PATENT APPLICATION

(11) **EP 0 689 817 A2**
(43) Date of publication of application: **03.01.1996**
(21) Application number: 95110168.2
(22) Date of filing: 29.06.1995
(51) Int. Cl.: A61F 13/15

(54) **Absorbent structure including an adhesive**

(30) Priority: 30.06.1994 US 269398
(71) Applicant: KIMBERLY-CLARK CORPORATION, Neenah, Wisconsin 54956 (US)
(72) Inventor: Chen, Franklin M.C., Appleton,WI 54914 (US); Gossen, Barbara Ann, Neenah, WI 54956 (US); Zenker, David Louis, Neenah, WI 54956 (US)
(74) Representative: Diehl, Hermann, Dr. Dipl.-Phys.

(57) **Abstract**

Disclosed is an absorbent structure including a wettable fibrous matrix, an adhesive, and a hydrogel-forming polymeric material, wherein the adhesive is substantially non-soluble and is substantially non-dispersible in a liquid to be contacted with the absorbent structure and the absorbent structure exhibits desired absorbent properties; disposable absorbent products, including the absorbent structure, intended for the absorption of body liquids; and a method for preparing the absorbent structure.

## Description

The present invention relates to an absorbent structure and a process of preparing same.

The use of hydrogel-forming polymeric materials, commonly known as superabsorbents, in disposable absorbent personal care products is known. Such absorbent materials are generally employed in absorbent products such as diapers, training pants, adult incontinence products, and feminine care products in order to increase the absorbent capacity of such products while reducing their overall bulk. Such absorbent materials are generally present in absorbent products in a fibrous matrix, such as a matrix of wood pulp fluff. A matrix of wood pulp fluff generally has an absorbent capacity of about 6 grams of liquid per gram of fluff. The absorbent materials described above generally have an absorbent capacity of at least about 10, preferably about 20, and often up to 100 times their weight in water. Clearly, incorporation of such absorbent materials in personal care products can reduce the overall bulk while increasing the absorbent capacity of such products.

A wide variety of materials have been described for use as hydrogel-forming polymeric materials in such personal care products. Such materials include natural-based materials such as agar, pectin, gums, carboxyalkyl starch, and carboxyalkyl cellulose, as well as synthetic materials such as polyacrylates, polyacrylamides, and hydrolyzed polyacrylonitrile.

It is often useful to try to adhere the hydrogel-forming polymeric materials within the fibrous matrix so as to keep the hydrogel-forming polymeric materials in its desired position. Such immobility will help to keep the hydrogel-forming polymeric materials more or less evenly dispersed within the fibrous matrix.

One method of initially immobilizing a hydrogel-forming polymeric material within a fibrous matrix is to moisten the fibrous matrix, deposit the hydrogel-forming polymeric material thereon, and then dry the fibrous matrix.

Another method of initially immobilizing a hydrogel-forming polymeric material within a fibrous matrix is to moisten the hydrogel-forming polymeric material so as to become substantially swollen with water, contact the swollen hydrogel-forming polymeric material with the fibrous matrix, and then dry the structure to remove most of the water and bond the hydrogel-forming polymeric material with the fibrous matrix.

Unfortunately, known methods of adhering a hydrogel-forming polymeric material to a fibrous matrix generally result in the absorbent structures having absorbent properties that are less than desired. When such absorbent materials are adhered to a fiber matrix with water or with known adhesives, for example, the absorbent properties of the hydrogel-forming polymeric material are generally negatively affected. Also, the bonding of the hydrogel-forming polymeric material to the fibrous matrix is generally not permanent, such that, when the absorbent structure is insulted with a liquid, the bonding breaks down to permit the hydrogel-forming polymeric material to lose contact with the fibrous matrix and migrate freely, such that the absorbent properties of the absorbent structure are negatively affected.

The present invention intends to overcome these problems. The object is solved by the absorbent structure according to independent claim 1, the disposable absorbent product of independent claim 2 and the process for preparing an absorbent structure according to independent claim 18.

Further advantages, features, aspects and details of the invention are evident from the dependent claims, the description and the accompanying drawings. The claims are intended to be understood as a first non-limiting approach of defining the invention in general terms.

The present invention relates to an absorbent structure including a fibrous matrix, an adhesive, and a hydrogel-forming polymeric material; disposable absorbent products, including the absorbent structure, intended for the absorption of body liquids; and a process for preparing the absorbent structure.

The present invention concerns an efficient and effective manner for preparing an absorbent structure comprising a fibrous matrix, an adhesive, and a hydrogel-forming polymeric material, wherein the hydrogel-forming polymeric material is effectively adhered to the fibrous matrix, and the absorbent properties of the hydrogel-forming polymeric material and the absorbent structure are substantially maintained when contacted with a liquid.

One aspect of the present invention concerns an absorbent structure comprising a fibrous matrix, an adhesive, and a hydrogel-forming polymeric material.

One embodiment of such an absorbent structure comprises a fibrous matrix, wherein the fibrous matrix is wettable with a liquid to be contacted with the absorbent structure; an adhesive contacting the fibrous matrix, wherein the adhesive is substantially non-soluble and is substantially non-dispersible in the liquid to be contacted with the absorbent structure; and a hydrogel-forming polymeric material contacting the adhesive; wherein the absorbent structure exhibits an Absorbency Under Load value of at least about 12 grams of liquid per gram of absorbent structure and a Capillary Tension Capacity value of at least about 6 grams of liquid per gram of absorbent structure.

In another aspect, the present invention concerns a disposable absorbent product for the absorption of liquids, such as body liquids.

One embodiment of such a disposable absorbent product comprises a liquid-permeable topsheet, a backsheet attached to the topsheet, and an absorbent structure positioned between the topsheet and the backsheet, wherein the absorbent structure comprises a fibrous matrix, wherein the fibrous matrix is wettable with a liquid to be contacted with the absorbent structure; an adhesive contacting the fibrous matrix, wherein the adhesive is substantially non-soluble and is substantially non-dispersible in the liquid to be contacted with the absorbent structure; and a hydrogel-forming polymeric material contacting the adhesive; wherein the absorbent structure exhibits an Absorbency Under Load value of at least about 12 grams of the liquid per gram of the absorbent structure, and wherein the absorbent structure exhibits a Capillary Tension Capacity value that is at least about 6 grams of the liquid per gram of the absorbent structure.

In another aspect, the present invention concerns a process for preparing an absorbent structure comprising a fibrous matrix, an adhesive, and a hydrogel-forming polymeric material.

One embodiment of such a process comprises contacting a fibrous matrix, wherein the fibrous matrix is wettable with a liquid to be contacted with the absorbent structure, with an adhesive, wherein the adhesive is substantially non-soluble and is substantially non-dispersible in the liquid to be contacted with the absorbent structure; and contacting the adhesive with a hydrogel-forming polymeric material, wherein the hydrogel-forming polymeric material becomes adhered to the fibrous matrix; wherein the absorbent structure exhibits an Absorbency Under Load value of at least about 12 grams of the liquid per gram of the absorbent structure and a Capillary Tension Capacity value that is at least about 6 grams of the liquid per gram of the absorbent structure.

Fig. 1 represents a disposable absorbent product according to the present invention.

Fig. 2 is an illustration of the equipment employed in determining the Absorbency Under Load (AUL) values.

Fig. 3 is a side elevational view of an apparatus used to measure Capillary Tension Capacity values of absorbent compositions at a negative pressure gradient and under an applied restraint.

Fig. 4 is a graph summarizing data from an Absorbency Under Load test on the absorbent structures of Example 1 according to the test methods described herein.

Fig. 5 is a graph summarizing data from a Capillary Tension Capacity test on the absorbent structures of Example 1 according to the test methods described herein.

As used herein, "hydrogel-forming polymeric material" is meant to refer to a high-absorbency material commonly referred to as a superabsorbent material. Such high-absorbency materials are generally capable of absorbing an amount of a liquid, such as synthetic urine, a 0.9 weight percent aqueous saline solution, or body liquids such as menses, urine, or blood, at least about 10, suitably about 20, and up to about 100 times the weight of the superabsorbent material at the conditions under which the superabsorbent material is being used. Typical conditions include, for example, a temperature of between about 0°C to about 100°C and suitably ambient conditions, such as about 23° C and about 30 to about 60 percent relative humidity. Upon absorption of the liquid, the superabsorbent material typically swells and forms a hydrogel.

The superabsorbent material may be formed from an organic hydrogel material, which may include natural materials, such as agar, pectin, and guar gum, as well as synthetic materials, such as synthetic hydrogel polymers. Synthetic hydrogel polymers include, for example, carboxymethyl cellulose, alkali metal salts of polyacrylic acid, polyacrylamides, polyvinyl alcohol, ethylene maleic anhydride copolymers, polyvinyl ethers, hydroxypropyl cellulose, polyvinyl morpholinone, polymers and copolymers of vinyl sulfonic acid, polyacrylates, polyacrylamides, and polyvinyl pyrridines. Other suitable hydrogel polymers include hydrolyzed acrylonitrile grafted starch, acrylic acid grafted starch, and isobutylene maleic anhydride copolymers, and mixtures thereof. The hydrogel polymers are preferably lightly crosslinked to render the material substantially water insoluble yet water swellable. Crosslinking may, for example, be by irradiation or covalent, ionic, Van der Waals, or hydrogen bonding. Suitable superabsorbent materials are typically available from various commercial vendors, such as The Dow Chemical Company, Hoechst Celanese, Allied Colloids Limited, or Stockhausen, Inc.

Suitably, the hydrogel-forming polymeric material is in the form of particles which, in the unswollen state, have maximum cross-sectional diameters within the range of from about 50 µm to about 1000 µm, preferably within the range of from about 100 µm to about 800 µm, as determined by sieve analysis according to American Society for Testing and Materials (ASTM) test method D-1921. It is understood that the particles of hydrogel-forming polymeric material falling within the ranges described above may comprise solid particles, porous particles, or may be agglomerated particles comprising many smaller particles agglomerated into particles falling within the described size ranges.

The hydrogel-forming polymeric material is present in the absorbent structure of the present invention in an amount effective to result in the absorbent structure being able to absorb a desired amount of liquid under desired conditions. The hydrogel-forming polymeric material is present in the absorbent structure of the present invention in an amount of beneficially from about 0.1 to about 20 grams, suitably from about 0.5 to about 15 grams, and more suitably from about 1 to about 10 grams. The hydrogel-forming polymeric material is beneficially present in the absorbent structure of the present invention in an amount of from about 5 to about 95 weight percent, suitably from about 10 to about 90 weight percent, and more suitably from about 20 to about 80 weight percent, based on the total weight of the hydrogel-forming polymeric material, fibrous matrix, and adhesive in the absorbent structure.

As used herein, the term "fiber" or "fibrous" is meant to refer to a particulate material wherein the length to diameter ratio of such particulate material is greater than about 10. Conversely, a "nonfiber" or "nonfibrous" material is meant to refer to a particulate material wherein the length to diameter ratio of such particulate material is about 10 or less.

A wide variety of natural and synthetic fibers can be employed in the preparation of the fibrous matrix of the present invention. Illustrative fibers include, but are not limited to, wood and wood products, such as wood pulp fibers, cellulose or cellulose acetate flocs, cotton linter flocs and the like, inorganic fibers, synthetic fibers such as nylon flocs, rayon flocs, polyacrylonitrile fibers, and the like.

It is also possible to use mixtures of one or more natural fibers, or one or more synthetic fibers, or combinations of the two. Preferred fibers are those which are wettable in nature. However, nonwettable fibers can also be used. It is possible to treat the fiber surfaces by an appropriate method to render them more or less wettable. When surface treated fibers are employed, the surface treatment is desirably nonfugitive; that is, the surface treatment desirably does not wash off the surface of the fiber with the first liquid insult or contact. For the purposes of this application, a surface treatment on a generally nonwettable polymer will be considered to be nonfugitive when a majority of the fibers demonstrate a liquid in air contact angle of less than 90° for three consecutive contact angle measurements, with drying between each measurement. That is, the same fiber is subjected to three separate contact angle determinations and, if all three of the contact angle determinations indicate a contact angle of liquid in air of less than 90°, the surface treatment on the fiber will be considered to be nonfugitive. If the surface treatment is fugitive, the surface treatment will tend to wash off of the fiber during the first contact angle measurement, thus exposing the nonwettable surface of the underlying fiber, and will demonstrate subsequent contact angle measurements greater than 90°. Beneficial wettability agents include polyakylene glycols, such as polyethylene glycols. The wettability agent is used in an amount comprising beneficially less than about 5 weight percent, suitably less than about 3 weight percent, and more suitably less than about 2 weight percent, of the total weight of the fiber, material, or absorbent structure being treated.

As used herein, the term "wettable" is meant to refer to a fiber or material which exhibits a liquid in air contact angle of less than 90°, wherein the liquid contacting the fiber or material is a liquid such as water, synthetic urine, urine, menses, blood, or a 0.9 weight percent aqueous saline solution. As used herein, the contact angle may be determined, for example, as set forth by Robert J. Good and Robert J. Stromberg, Ed., in "Surface and Colloid Science - Experimental Methods", Vol. 11, (Plenum Press, 1979). Suitably, a wettable fiber refers to a fiber which exhibits a 0.9 weight percent aqueous saline solution in air contact angle of less than 90°, at a temperature between about 0°C and about 100°C, and suitably at ambient conditions, such as about 23°C.

Fibrous matrixes for incorporation into an absorbent structure are generally well known. A fibrous matrix may take the form of, for example, a batt of comminuted wood pulp fluff, a tissue layer, a hydroentangled pulp sheet, a mechanically softened pulp sheet, or of a web structure comprising an entangled fibrous mass formed, for example, from an extruded thermoplastic composition. Suitably, the fibrous matrix is formed so as to constrain or entrap the hydrogel-forming polymeric material within, or onto, its structure. The hydrogel-forming polymeric material may be incorporated into or onto the fibrous matrix either during or after the formation of the general form of the fibrous matrix.

Suitably, the fiber matrix has a basis weight ranging from about 0.025 grams per square meter of fiber matrix material to about 400 grams per square meter of fiber matrix material.

Suitably, the fiber matrix has a density ranging from about 0.05 grams per cubic centimeter of fiber matrix material to about 0.5 grams per cubic centimeter of fiber matrix material.

Suitably, the fibers comprising the fiber matrix have a fiber length ranging from about 0.1 centimeter to about 3.0 centimeters.

The fibrous matrix useful in the present invention may be formed by an air-laying process, a spunbond or meltblown process, a carding process, a wet-laid process, or by essentially any other process, known to those skilled in the art, for forming a fibrous matrix.

The fibrous matrix may be in the form of a single, integrally formed layer or of a composite comprising multiple layers. If the fibrous matrix comprises multiple layers, the layers are preferably in liquid communication with one another such that a liquid present in one fibrous layer can flow or be transported to the other fibrous layer. For example, the fibrous layers may be separated by cellulosic tissue wrap sheets known to those skilled in the art.

The hydrogel-forming polymeric material may be distributed in the individual layers in a generally uniform manner or may be present in the fibrous layers as a layer or other nonuniform distribution.

When the fibrous matrix comprises a single, integrally formed layer, the concentration of hydrogel-forming polymeric material may increase along the thickness of the fibrous matrix in a gradual, nonstepwise fashion or in a more stepwise fashion. Similarly, the density may decrease through the thickness in a nonstepwise manner or in a stepwise manner.

The fibrous matrix is beneficially present in the absorbent structure of the present invention in an amount of from about 5 to about 95 weight percent, suitably from about 10 to about 90 weight percent, and more suitably from about 20 to about 80 weight percent, based on the total weight of the hydrogel-forming polymeric material, fibrous matrix, and adhesive in the absorbent structure.

It has been discovered that, by using specific adhesive materials to adhere the hydrogel-forming polymeric material to the fibrous matrix, the hydrogel-forming polymeric material is more effectively and permanently adhered to the fibrous matrix. As such, the absorbent properties of the absorbent structure are improved as compared to when other adhesives or methods are used to adhere the hydrogel-forming polymeric material to the fibrous matrix.

One advantage to more permanently adhering the hydrogel-forming polymeric material to the fibrous matrix is that the hydrogel-forming polymeric material will better stay in its desired location. This helps to provide the desired absorbent properties, prevent dry or gelled hydrogel-forming polymeric material from transferring to the inner liner of an absorbent product or to a wearer's skin, and to prevent housekeeping or cleaning problems caused by the hydrogel-forming polymeric material separating from the absorbent product. Another advantage to more permanently adhering the hydrogel-forming polymeric material to the fibrous matrix is that the hydrogel-forming polymeric material remains in contact with the fibrous matrix so as to take advantage of the liquid transport capability of the fibrous matrix which more fully utilizes the absorbent capacity of the hydrogel-forming polymeric material and the absorbent structure.

The adhesive useful in the present invention is substantially non-soluble and non-dispersible in the liquid to be contacted with the absorbent structure comprising the adhesive. Such liquids include water, a 0.9 weight percent aqueous saline solution, synthetic urine, and body liquids such as urine, menses, and blood. Because the adhesives useful in the present invention are substantially non-soluble and non-dispersible in the liquid to be contacted with the absorbent structure comprising the adhesive, the absorbent properties of the absorbent structure will not be substantially negatively-affected when the absorbent structure is contacted with the liquid. This is generally in contrast to the use of an adhesive which is substantially soluble or dispersible in the liquid to be contacted with the absorbent structure since, upon contact of the liquid with the substantially soluble or dispersible adhesive, such adhesive will become substantially soluble or dispersed within the liquid so as to substantially no longer adhere the hydrogel-forming polymeric material to the fibrous matrix. It is also believed that, when the hydrogel-forming polymeric material is no longer substantially adhered to the fibrous matrix, the beneficial interactions between the hydrogel-forming polymeric material and the fibrous matrix will be negatively affected. Thus, because the adhesive of the present invention is substantially non-soluble and substantially non-dispersible in the liquid to be contacted with the absorbent structure, the hydrogel-forming polymeric material cannot be as easily detached from the fibrous matrix as compared to when a substantially soluble or dispersible adhesive is used. Furthermore, when an adhesive becomes substantially soluble or dispersed within a liquid contacting the absorbent structure, the soluble adhesive fragments may interfere with the ability of the hydrogel-forming polymeric material to absorb the liquid as, for example, when under an applied load.

As used herein, the term "substantially non-soluble" is meant to represent that substantially no soluble fraction of the adhesive can be detected in a liquid contacted with the adhesive by such known laboratory techniques as intrinsic viscosity measurements or light scattering experiments, such as those described in "Principles of Colloid and Surface Chemistry", by Paul Hiemenz (1977), incorporated herein by reference.

As used herein, the term "substantially non-dispersible" is meant to represent that, when contacted with a liquid, substantially no dispersible fraction of the adhesive material, within a size distribution range of about 1 µm to about 100 µm, can be filtrated out of the liquid by using conventional filter paper.

The adhesives useful in the present invention should have low levels of extractable fractions in the liquid to be contacted with the absorbent structure, wherein the adhesive extractable fraction does not substantially effect the absorbent properties of the absorbent structure. As used herein, the term "extractable fractions" is meant to represent the soluble and dispersible fractions of an adhesive material. In general, extractable fractions from an adhesive will have a negative effect on the absorbent properties of the hydrogel-forming polymeric material and the absorbent structure, because the extractable fractions generally interfere with the ability of the hydrogel-forming polymeric material or the absorbent structure to absorb a liquid. In general, the effects of adhesive extractable fraction levels can be quantified, for example, by measuring the Absorbency Under Load (AUL) value and the Capillary Tension Capacity (CTC) value (both tests of which are described herein) of samples of the absorbent structure. The adhesive of the present invention has an extractable fraction level that is beneficially less than about 0.1, suitably less than about 0.05, and more suitably less than about 0.01 weight percent of the total weight of adhesive applied to an absorbent structure.

Adhesives useful in the present invention may be of any known type, such as a thermoplastic hot-melt adhesive, a reactive adhesive, or the like. An example of a thermoplastic hot-melt adhesive includes a synthetic rubber-based adhesive based on polystyrene-polybutadiene-polystyrene chemistry and a tackifier based on hydrocarbon chemistry, available from the National Starch Corporation under the trade designation NS 34-5541. A description of compositions of hot-melt adhesives can be found, for example, in "CRC Elastomer Technology Handbook", edited by Nicholas P. Cheremisinoff (CRC Press, 1993), Chapter 24, incorporated herein by reference.

Examples of reactive adhesives include crosslinked amine-epoxide compounds or moisture-cured polyurethanes. The chemistry of such reactive adhesives is known to those skilled in the art and may be found, for example, in "Contemporary Polymer Chemistry", by Harry Alcock and Frederick Lampe (Prentice Hall, 1990), incorporated herein by reference.

The adhesive used in the present invention may be either wettable or non-wettable with a liquid to be contacted with the absorbent structure. Generally, the lower limit of adhesive application coverage is determined by the minimum amount of adhesive needed to adhere an effective amount of hydrogel-forming polymeric material to the fibrous matrix so as to achieve the desired absorbent properties. If the adhesive application coverage is too low, the amount of hydrogel-forming polymeric material that may be adhered to the fibrous matrix will be ineffective to substantially improve the absorbent properties of the absorbent structure.

Generally, the upper limit of adhesive application coverage is determined by the surface wettability of the adhesive. If the adhesive is nonwettable and the surface application coverage of the adhesive is too high, the surface of the fibrous matrix could be made too nonwettable to be an effective absorbent structure, since fluid transport properties would be negatively affected. As such, relatively higher application coverage levels of a wettable adhesive could generally be tolerated as compared to a nonwettable adhesive. If the adhesive is wettable, the adhesive is suitably applied to an outer surface of the fibrous matrix in an amount ranging from about 0.00016 gram of adhesive per square cm of fibrous matrix (about 0.001 gram of adhesive per square inch of fibrous matrix) to about 0.016 gram of adhesive per square centimeter of fibrous matrix (0.1 gram of adhesive per square inch of fibrous matrix). If the adhesive is nonwettable, the adhesive is suitably applied to a surface of the fibrous matrix in an amount ranging from about 0.00016 gram of adhesive per square centimeter of fibrous matrix (about 0.001 gram of adhesive per square inch of fibrous matrix) to about 0.008 gram of adhesive per square centimeter of fibrous matrix (about 0.05 gram of adhesive per square inch of fibrous matrix).

The adhesive is beneficially present in the absorbent structure of the present invention in an amount of from greater than about 0.5 to about 30 weight percent, suitably from about 1 to about 25 weight percent, and more suitably from about 1 to about 10 weight percent, based on the total weight of the hydrogel-forming polymeric material, fibrous matrix, and adhesive in the absorbent structure.

A more uniform dispersal of the adhesive will typically result in less of the adhesive being needed in order to achieve an effective and efficient adhesion of the hydrogel-forming polymeric material onto a fibrous matrix in an absorbent structure as compared to where the adhesive is not as uniformly dispersed onto the fibrous matrix. In addition, the upper and lower limits of the amount of adhesive that is to be used in the absorbent structure may be affected by the nature of the fibrous matrix and the hydrogel-forming polymeric material. Generally speaking, one would like to use as little of the adhesive as possible.

The adhesive may also be applied to the fibrous matrix in a nonuniform pattern so as to adhere the hydrogel-forming polymeric material to the fibrous matrix in a nonuniform pattern. As such, zones of higher and lower hydrogel-forming polymeric material concentration may be created by selectively controlling the adhesive pattern and adhesive amount. For example, more adhesive could be applied in a pattern in a front absorbent panel of a diaper to provide acceptable performance specific to males. Alternatively, more adhesive could be applied to higher stress regions, such as the crotch of a diaper, so as to keep the hydrogel-forming polymeric material adhered to the fibrous matrix in that region.

Methods of incorporating the hydrogel-forming polymeric material into the fibrous matrix are known to those skilled in the art. Suitable methods include incorporating the hydrogel-forming polymeric material into the matrix during formation of the matrix, such as by air-laying the fibers of the fibrous matrix and the hydrogel-forming polymeric material at the same time or wet-laying the fibers of the fibrous matrix and the hydrogel-forming polymeric material at the same time. Alternatively, it is possible to apply the hydrogel-forming polymeric material to the fibrous matrix after formation of the fibrous matrix. Other methods include sandwiching the hydrogel-forming polymeric material between two sheets of material, at least one of which is fibrous and liquid permeable. The hydrogel-forming polymeric material may be generally uniformly located between the two sheets of material or may be located in discrete pockets formed by the two sheets. Additionally, the hydrogel-forming polymeric material may be located on both the top and the bottom of a single fibrous matrix by applying the adhesive to both the top and the bottom of the fibrous matrix and then contacting the hydrogel-forming polymeric material with both layers of adhesive.

The hydrogel-forming polymeric material, the adhesive, and the fibrous matrix may generally be mixed or combined in any acceptable manner, in order to prepare the absorbent structure of the present invention, as long as the absorbent structure exhibits the desired absorbent and other properties described herein.

One embodiment of such a process comprises contacting a fibrous matrix with an adhesive and then contacting the adhesive with a hydrogel-forming polymeric material. The adhesive is suitably spread onto an outer surface of the fibrous matrix and then the hydrogel-forming polymeric material is sprinkled or dispersed over the same outer surface of the fibrous matrix so as to come into contact with and adhere to the adhesive so as to become adhered to the fibrous matrix. Optionally, a second fibrous matrix may then be layered onto the first fibrous matrix so as to sandwich the adhesive and hydrogel-forming polymeric material between the two fibrous matrixes.

The adhesive may be contacted first with the fibrous matrix by known methods, such as by spraying, printing, applying as a dry particulate and then applying heat to melt the adhesive, and the like, or may be first applied to the hydrogel-forming polymeric material by known methods such as by spraying, application by a fluidized bed, and the like. The hydrogel-forming polymeric material may be contacted with the adhesive and fibrous matrix by known methods, such as by use of vacuum or gravimetric processes.

One such method includes using a vacuum process to prepare a layered absorbent structure comprising a tissue web base and a hydrogel-forming polymeric material layer ideally located between two fibrous matrix layers. A tissue web base moves over a vacuum drum former which attracts the desired fibrous material, adhesive, and hydrogel-forming polymeric material from a forming chamber.

Another method of incorporating a hydrogel-forming polymeric material into an absorbent structure includes using a gravimetric process wherein the hydrogel-forming polymeric material is gravimetrically applied to a moving fibrous matrix with adhesive applied on an outer surface of the fibrous matrix.

It has been found that, when an adhesive as described herein is used to prepare the absorbent structure of the present invention, the absorbent structure exhibits superior absorbent properties as compared to an otherwise essentially identical absorbent structure that is not prepared using the adhesive but, for example, is prepared using a different adhesive or moisture to adhere the hydrogel-forming polymeric material to the fibrous matrix.

Beneficially, the absorbent structure of the present invention exhibits improved absorbent properties, such as Absorbency Under Load values and Capillary Tension Capacity values, when prepared using the adhesive described herein, as compared to using other adhesives or methods to initially adhere the hydrogel-forming polymeric material to the fibrous matrix.

The hydrogel-forming polymeric material and the absorbent structure employed in the absorbent products of the present invention suitably should be able to absorb a liquid under an applied load. For the purposes of this application, the ability of an absorbent structure to absorb a liquid under an applied load and thereby perform work is quantified as the Absorbency Under Load (AUL) value. The AUL value is expressed as the amount (in grams) of an aqueous 0.9 weight percent sodium chloride solution which the absorbent structure can absorb in 60 minutes per gram of absorbent structure under a load of approximately 6.0 kPa (about 0.9 pound per square inch) while restrained from swelling in the plane normal to the applied load. The absorbent structure of the present invention beneficially exhibits an AUL value of at least about 12, more beneficially of at least about 15, suitably of at least about 20, more suitably of at least about 25, and up to about 50 grams of liquid per gram of absorbent structure. The method by which the AUL value may be determined is set forth in the Test Methods section herein or, for example, in detail in US-A-5,149,335 or US-A-5,247,072, incorporated herein by reference.

The absorbent structure of the present invention exhibits an Absorbency Under Load value, as described in the Test Methods section herein, that is beneficially at least about 10 percent greater, suitably at least about 25 percent greater, and more suitably at least about 50 percent greater than the Absorbency Under Load value exhibited by an otherwise identical absorbent structure which does not comprise the adhesive.

The absorbent structure of the present invention exhibits a Capillary Tension Capacity value, at a negative pressure gradient of about 15 centimeters and under a load of 3.6 kPa (about 0.54 pound per square inch) and for a time period of about 1000 seconds, as described in the Test Methods sections herein, beneficially of at least about 6, more beneficially of at least about 8, suitably of at least about 10, and more suitably of at least about 12, and up to about 25 grams of liquid per gram of absorbent structure.

The absorbent structure of the present invention exhibits a Capillary Tension Capacity value, at a negative pressure gradient of about 15 centimeters and under a load of about 3.6 kPa (about 0.54 pound per square inch) and for a time period of about 1000 seconds, that is beneficially at least about 50 percent greater, suitably at least about 100 percent greater, and more suitably at least about 200 percent greater, than the Capillary Tension Capacity value exhibited by an otherwise identical absorbent structure which does not comprise the adhesive.

The absorbent structures according to the present invention are suited to absorb many liquids, such as water, saline, synthetic urine, and body liquids, such as urine, menses, and blood, and are suited for use in disposable absorbent products, such as diapers, adult incontinent products, and bed pads; in catamenial devices, such as sanitary napkins, and tampons; and in other absorbent products, such as wipes, bibs, wound dressings, and surgical capes or drapes. Accordingly, in another aspect, the present invention relates to a disposable absorbent product comprising an absorbent structure as described herein.

Use of the described absorbent structures in disposable absorbent products allows for the formation of a disposable absorbent product which is able to rapidly receive a discharged liquid and yet which product is thin. Such disposable absorbent products generally comprise a liquid-permeable topsheet, a backsheet attached to the topsheet, and an absorbent structure, such as an absorbent structure of the present invention, located between the topsheet and backsheet.

Exemplary disposable absorbent products are generally described in US-A-4,710,187; US-A-4,762,521; US-A-4,770,656; US-A-4,798,603; and U.S. Serial No. 08/096,654, filed July 22, 1993, in the name of Hansen et al., which references are incorporated herein by reference.

In one embodiment of the present invention, a disposable absorbent product is provided, which disposable absorbent product comprises a liquid-permeable topsheet, a backsheet attached to the topsheet, and an absorbent structure positioned between the topsheet and the backsheet, wherein the absorbent structure comprises a wettable fibrous matrix, an adhesive contacting the fibrous matrix, wherein the adhesive is substantially non-soluble and is substantially non-dispersible in a liquid to be contacted with the absorbent structure, and a hydrogel-forming polymeric material contacting the adhesive, wherein the absorbent structure exhibits desired absorbent properties.

While one embodiment of the invention will be described in terms of the use of an absorbent structure in an infant diaper, it is to be understood that the absorbent structure is equally suited for use in other disposable absorbent products known to those skilled in the art.

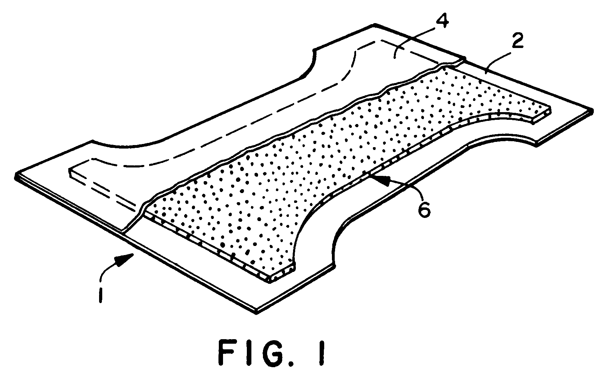

Turning now to the drawings, Fig. 1 illustrates a disposable diaper 1 according to one embodiment of the present invention. Disposable diaper 1 includes a backsheet 2, a topsheet 4, and an absorbent structure 6, located between the backsheet 2 and the topsheet 4. Absorbent structure 6 is an absorbent structure according to the present invention. Specifically, in the illustrated embodiment, absorbent structure 6 comprises a fibrous matrix, an adhesive, and a hydrogel-forming polymeric material.

Those skilled in the art will recognize materials suitable for use as the topsheet and backsheet. Exemplary of materials suitable for use as the topsheet are liquid-permeable materials, such as spunbonded polypropylene or polyethylene having a basis weight of from about 15 to about 25 grams per square meter. Exemplary of materials suitable for use as the backsheet are liquid-impervious materials, such as polyolefin films, as well as vapor-pervious materials, such as microporous polyolefin films.

The absorbent structures of the present invention may generally be of any size or dimension as long as the absorbent structure exhibits the desired absorbent characteristics as described herein. Typically, the absorbent structures will have a volume of at least about 18 cubic centimeters, such as with a width of about 6 centimeters, a length of about 6 centimeters, and a depth of about 0.5 centimeter. Suitably, the absorbent structure will have a volume of at least about 60 cubic centimeters, such as with a width of about 10 centimeters, a length of about 6 centimeters, and a depth of about 1 centimeter.

The absorbent structure of the present invention may also be used or combined with other absorbent structures, with the absorbent structure of the present invention being used as a separate layer or as an individual zone or area within a larger, composite absorbent structure. The absorbent structure of the present invention may be combined with other absorbent structures by methods well known to those skilled in the art, such as by using adhesives or simply by layering the different structures together and holding together the composite structures with, for example, tissue. Additionally, different types of hydrogel-forming polymeric material could be located in different layers or zones of an absorbent structure.

Absorbent products and structures according to all aspects of the present invention are generally subjected, during use, to multiple insults of a body liquid. Accordingly, the absorbent products and structures are desirably capable of absorbing multiple insults of body liquids in quantities to which the absorbent products and structures will be exposed during use. The insults are generally separated from one another by a period of time.

### Test Methods

### Absorbency Under Load

The Absorbency Under Load (AUL) is a test which measures the ability of an absorbent material or structure to absorb a liquid (0.9 weight percent solution of sodium chloride in distilled water) while under an applied load or restraining force of about 6 kPa (about 0.9 pound per square inch) for a time period of about 60 minutes.

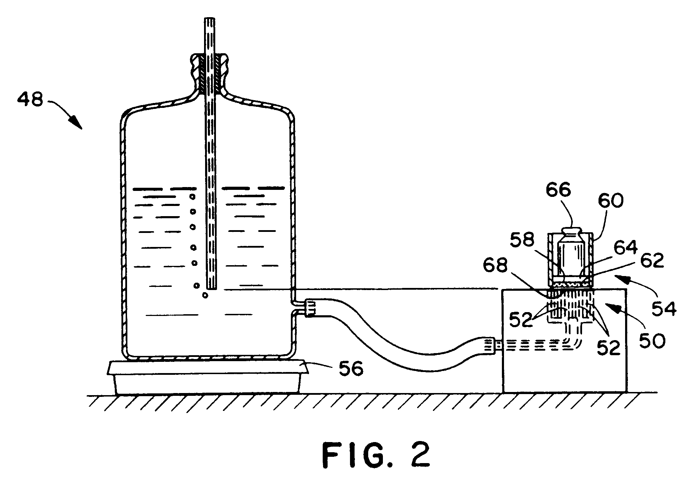

The ability of an absorbent structure to absorb a liquid while under load is determined as follows. Referring to Fig. 2, a demand absorbency tester (DAT) 48 is used, which is similar to a GATS (gravimetric absorbency test system) available from M/K Systems, Danners, MA, as well as the system described by Lichstein at pages 129-142 of the INDA Technological Symposium Proceedings, March 1974. A porous plate 50 is used, having ports 52 confined within a 2.5 centimeter diameter area and covered by the absorbency under load (AUL) apparatus 54. An electrobalance 56 is used to measure the flow of fluid into the absorbent structure 58. For this test, the fluid employed is an aqueous solution containing 0.9 weight percent sodium chloride used at room temperature (^{∼}23°C).

The special AUL apparatus 54 used to contain the absorbent structure comprises a cylinder 60 made from 2.54 cm (1 inch) inside diameter thermoplastic tubing which is machined-out slightly to be sure of concentricity. A 100 mesh stainless steel wire cloth 62 is adhered to the bottom of cylinder 60. Care must be taken to maintain a flat, smooth bottom and not distort the inside of the cylinder. A 4.4 gram piston 64 is made from 1 inch diameter solid material (e.g., Plexiglas™) and is machined to closely fit without binding in the cylinder 60. A standard 300 gram weight 66 is used to provide about 6 kPa (about 0.9 pound per square inch) restraining load. A circular, 2.54 cm (1 inch) diameter sample of absorbent structure is utilized for testing AUL.

This test is initiated by placing a three centimeter diameter GF/A glass filter paper 68 onto the plate 50. The paper is sized to be larger than the internal diameter and smaller than the outside diameter of the cylinder 60 to ensure good contact, while eliminating evaporation over the ports 52 of the DAT 48, and then allowing saturation to occur. The absorbent structure 58 is weighed and placed on the wire cloth 62 at the bottom of the AUL apparatus 54. After carefully placing, without pressing, the piston 64 and weight 66 on the absorbent structure 58 in the cylinder 60, the AUL apparatus 54 is placed on the glass filter paper 68. The amount of fluid picked up is monitored as a function of time, either directly by hand, with a strip-chart recorder or directly into a data acquisition or personal computer system.

The amount (in grams) of fluid picked up after 60 minutes, divided by the weight of the sample, is the AUL value in grams of picked up fluid per gram of absorbent structure (g/g). The rate of fluid pick up can also be measured. Two checks can be made to ensure the accuracy of the instantaneous final read-out. First, the height the piston 64 rises, multiplied by the cross-sectional area of the cylinder 60, should nearly equal the amount of fluid picked up. Second, the AUL apparatus 54 can be weighed before and after the test, and the difference in weight should nearly equal the fluid picked up.

### Capillary Tension Capacity Test

The Capillary Tension Capacity (CTC) Test is a test which measures the ability of an absorbent structure to absorb a liquid (0.9 weight percent solution of sodium chloride in distilled water) while under an applied load or restraining force of about 3.6 kPa (about 0.54 pound per square inch) while subjected to a negative pressure gradient of about 15 centimeters and for a time of about 1000 seconds.

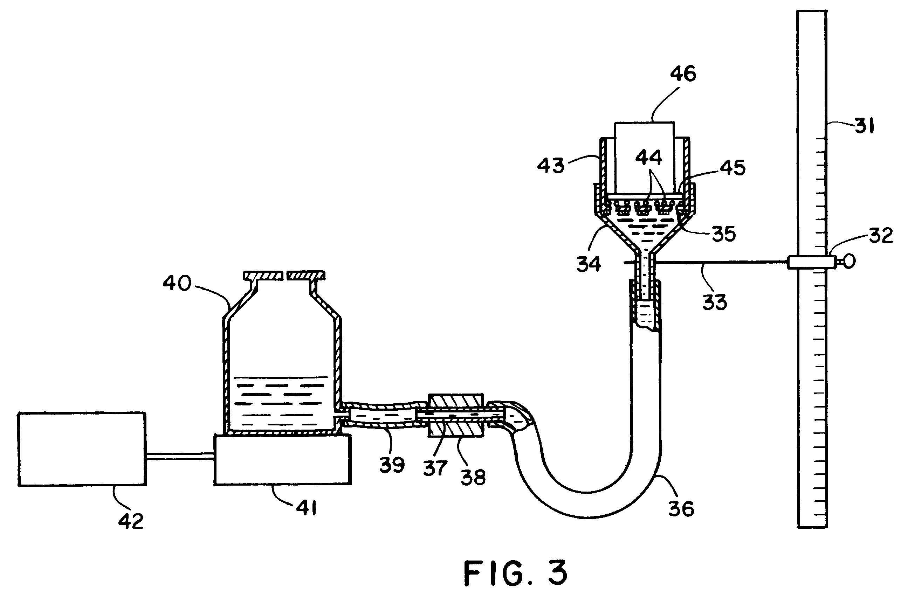

Referring to Fig. 3, the apparatus and method for determining CTC values will be described. Shown is a perspective view of the apparatus in position during a test.

Shown is a laboratory stand 31 which is gradated in centimeters and which has an adjustable collar 32 for raising and lowering a support ring 33. The support ring 33 supports a funnel 34 which has a 6 centimeter diameter. In the funnel 34, is placed a porous glass plate 35 which has a nominal maximum pore diameter of about 40 to 60 µm. To the bottom of the funnel 34, is attached a first flexible plastic tubing 36, which is attached at its other end to a rigid plastic tube 37, which is held in place with a clamp 38. The other end of the rigid plastic tube 37 is attached to a second flexible plastic tubing 39 which is attached at its other end to a vented liquid reservoir 40. The vented liquid reservoir 40 rests on a balance 41, which is attached to a recorder 42, which is used to record the weight loss of liquid from the vented liquid reservoir 40 as liquid is absorbed by the sample being evaluated.

A plastic sample cup 43, which contains the absorbent structure sample 44 to be tested, has a liquid-permeable bottom and rests on top of the porous glass plate 35 in the funnel 34. A weight 46 rests on top of a spacer disc 45, resting on top of the absorbent structure sample.

The sample cup 43 consists of a plastic cylinder having a 2.54 cm (1 inch) inside diameter and an outside diameter of 3.175 cm (1.25 inches). The bottom of the sample cup 43 is formed by adhering a 100 mesh metal screen, having 150 µm openings, to the end of the cylinder by heating the screen above the melting point of the plastic and pressing the plastic cylinder against the hot screen to melt the plastic and bond the screen to the plastic cylinder.

To carry out the test, a 0.160 gram sample of the absorbent structure 44 is placed into the sample cup. The sample is then covered with a plastic spacer disc 45, weighing 4.4 grams, which is slightly smaller than the inside diameter of the sample cup and serves to protect the sample from being disturbed during the test. A 183.52 gram weight 46 is then placed on top of the spacer disc, thereby applying a load of about 3.6 kPa (about 0.54 pound per square inch). The sample cup is placed on the porous glass plate 35.

A negative pressure gradient is established by lowering the funnel 34 until liquid from the vented liquid reservoir 40 flows through the tubing 37, 38, and 39 into the funnel 34 to make contact with the porous glass plate 35. The funnel 34 is then raised to a level along the gradated laboratory stand 31 until a desired negative pressure gradient (as measured in difference in height, in centimeters, between the top of the liquid level in the vented liquid reservoir 40 and the level of the porous glass plate 35) of about 15 centimeters is established. The recorder is then allowed to measure the amount of liquid which is removed from the vented liquid reservoir 40 and absorbed by the absorbent structure, on a gram of liquid absorbed per gram of absorbent material basis, over a period of time, such as about 1000 seconds.

### Example 1

Absorbent structures were prepared. A fibrous matrix comprising a barrier tissue having a cross section width of about 10.16 cm (about 4 inches) and a basis weight of about 11.87 g/m² (0.35 ounce per square yard) was used. Different adhesives were either sprayed or knife-coated onto a surface of the barrier tissue. A hydrogel-forming polymeric material, a sodium polyacrylate available from Stockhausen, Inc. under the trade designation FAVOR 870, was sieved to obtain particles having a particle size range of between about 3x10⁻⁴ meter to about 6x10⁻⁴ meter. The hydrogel-forming polymeric material particles were then sprinkled onto the adhesive-coated surface of the barrier tissue to form a substantially uniform monolayer.

The adhesives evaluated in this example and the amount and method of applying them to the tissue barrier were as follows:
Adhesive 1: A hot-melt construction adhesive, available from the National Starch Corporation under the trade designation NS 34-5541. Applied by spraying in an amount of about 0.004 g/cm² (about 0.025 gram per square inch) of barrier tissue.
Adhesive 2: The same as Adhesive 1 except that it was applied to the barrier tissue in an amount of about 0.008 g/cm² (about 0.050 gram per square inch) of barrier tissue.
Adhesive 3: A hot-melt adhesive comprising a water-soluble polyethylene oxide grafted polyvinyl alcohol, available from the National Starch Corporation under the trade designation CYCLOFLEX. Applied by spraying in an amount of about 0.004 g/cm² (about 0.025 gram per square inch) of barrier tissue.
Adhesive 4: A hot-melt adhesive comprising a water-soluble polyvinyl methylether, available from Findley Adhesives, Inc. under the trade designation E-42. Applied by spraying in an amount of about 0.004 g/cm² (about 0.025 gram per square inch) of barrier tissue.
Adhesive 5: A polyvinyl-alcohol-based latex adhesive, available from the National Starch Corporation under the trade designation NS 33-9156. Applied by spraying in an amount of about 0.002 g/cm² (about 0.01 gram per square inch) of barrier tissue.
Adhesive 6: An aqueous dispersion comprising a microcrystalline cellulose, a depolymerized alpha-cellulose, available from the FMC Corporation under the trade designation RC-591. Applied by hand with a knife in an amount of about 0.002 g/cm² (about 0.01 gram per square inch) of barrier tissue.

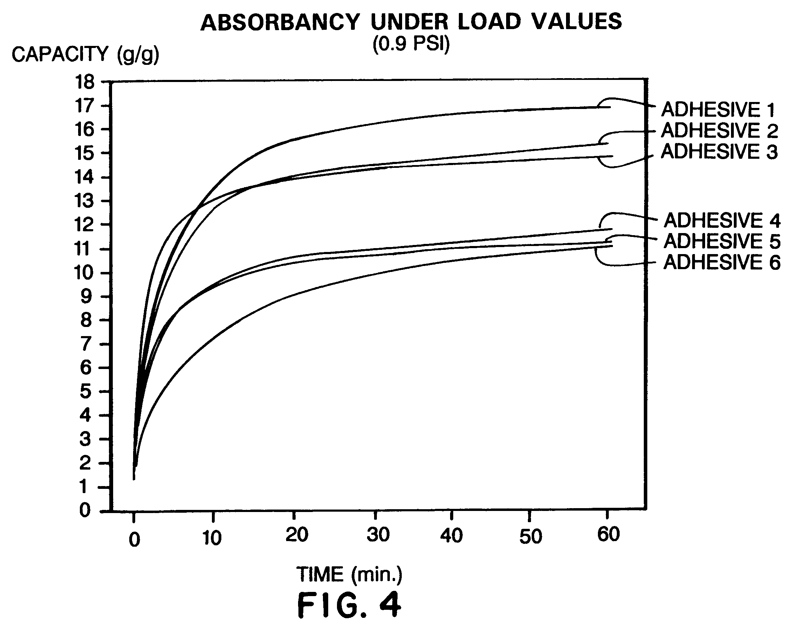

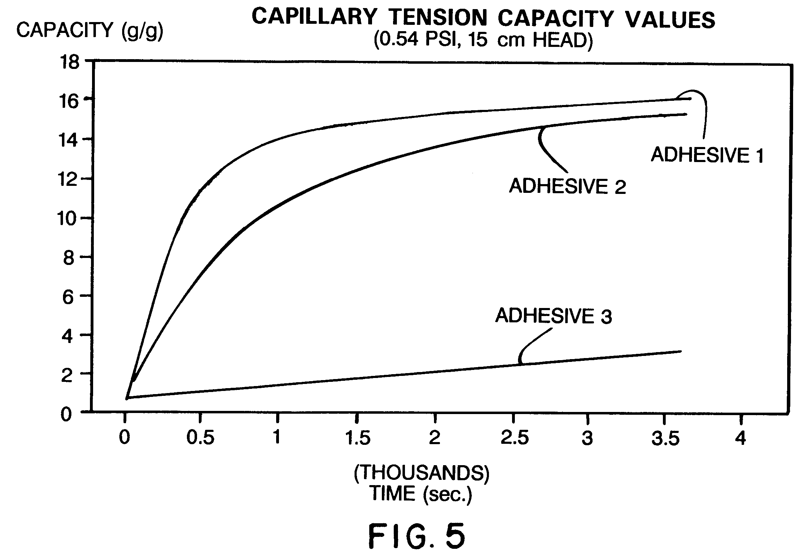

The prepared absorbent structures were die-cut into circular samples having a diameter of about 2.54 cm (about 1 inch). The samples were then evaluated for Capillary Tension Capacity values and Absorbency Under Load values. The results are shown in Figure 4 and Figure 5.

From Figure 4, it can be seen that the Absorbency Under Load value was dependent on the type of adhesive used. In liquids such as a 0.9 weight percent saline solution or bodily fluids, Adhesives 1 and 2 are not substantially soluble, Adhesives 3, 4, and 5 are substantially soluble, and Adhesive 6 is substantially dispersible but substantially non-soluble. As such, Adhesives 1 and 2 did not substantially have any extractable fractions with which to adversely affect the performance of the hydrogel-forming polymeric material.

From Figure 5, it can be seen that the Capillary Tension Capacity value was dependent on the type of adhesive used. Substantially insoluble Adhesive 1 produced much better results than substantially soluble Adhesive 4 and quicker results than substantially dispersible Adhesive 6. The slower liquid intake of the sample using Adhesive 6, as compared to the sample using Adhesive 1, is believed to be due to the detachment of Adhesive 6 from the barrier tissue which results in less interaction between the fibers of the barrier tissue and the hydrogel-forming polymeric material particles.

Those skilled in the art will recognize that the present invention is capable of many modifications and variations without departing from the scope thereof. Accordingly, the detailed description and examples set forth above are meant to be illustrative only and are not intended to limit, in any manner, the scope of the invention as set forth in the appended claims.

## Claims

1. An absorbent structure (6,58) comprising:
a fibrous matrix, wherein the fibrous matrix is wettable with a liquid to be contacted with the absorbent structure;
an adhesive contacting the fibrous matrix, wherein the adhesive is substantially non-soluble and is substantially non-dispersible in the liquid to be contacted with the absorbent structure; and
a hydrogel-forming polymeric material contacting the adhesive; wherein the absorbent structure exhibits an Absorbency Under Load value of at least about 12 grams of the liquid per gram of the absorbent structure, and wherein the absorbent structure exhibits a Capillary Tension Capacity value that is at least about 6 grams of the liquid per gram of the absorbent structure.

2. A disposable absorbent product (1) comprising a liquid-permeable topsheet (4), a backsheet (2) attached to the liquid-permeable topsheet (4), and an absorbent structure (6) positioned between the liquid-permeable topsheet (4) and the backsheet (2), wherein the absorbent structure (6) comprises
a fibrous matrix, wherein the fibrous matrix is wettable with a liquid to be contacted with the absorbent structure (6);
an adhesive contacting the fibrous matrix, wherein the adhesive is substantially non-soluble and is substantially non-dispersible in the liquid to be contacted with the absorbent structure (6); and
a hydrogel-forming polymeric material contacting the adhesive;
wherein the absorbent structure (6) exhibits an Absorbency Under Load value of at least about 12 grams of the liquid per gram of the absorbent structure (6), and wherein the absorbent structure (6) exhibits a Capillary Tension Capacity value that is at least about 6 grams of the liquid per gram of the absorbent structure (6).

3. The absorbent structure of claim 1 or 2 wherein the hydrogel-forming polymeric material is able to absorb an amount of 0.9 weight percent solution of sodium chloride in water at least about 10 times the weight of the hydrogel-forming polymeric material.

4. The absorbent structure of one of the preceding claims wherein the hydrogel-forming polymeric material is selected from the group consisting of agar, pectin, and guar gum, carboxymethyl cellulose, alkali metal salts of polyacrylic acid, polyacrylamides, polyvinyl alcohol, ethylene maleic anhydride copolymers, polyvinyl ethers, hydroxypropyl cellulose, polyvinyl morpholinone, polymers and copolymers of vinyl sulfonic acid, polyacrylates, polyacrylamides, and polyvinyl pyrridines.

5. The absorbent structure of one of the preceding claims wherein the absorbent structure (6,58) comprises from about 5 to about 95 weight percent hydrogel-forming polymeric material, from about 5 to about 95 weight percent fibrous matrix, and from about 0.5 to about 30 weight percent adhesive, based on total weight of the hydrogel-forming polymeric material, fibrous matrix, and the adhesive in the absorbent structure.

6. The absorbent structure of one of the preceding claims wherein the fiber matrix comprises fibers selected from the group consisting of wood pulp, cellulose, cellulose acetate, cotton linter, nylon, rayon, and polyacrylonitrile fibers.

7. The absorbent structure of one of the preceding claims wherein the fibrous matrix is in the form selected from the group consisting of a batt of comminuted wood pulp fluff, a tissue layer, a hydroentangled pulp sheet, a mechanically softened pulp sheet, and a web structure.

8. The absorbent structure of one of the preceding claims wherein the adhesive is selected from the group consisting of thermoplastic hot-melt adhesives and reactive adhesives, preferably consisting of synthetic rubber-based adhesives based on polystyrene-polybutadiene-polystyrene chemistry, crosslinked amine-epoxide compounds, and moisture-cured polyurethanes.

9. The absorbent structure of one of claims 1 to 8 wherein the adhesive is wettable with the liquid to be contacted with the absorbent structure.

10. The absorbent structure of claim 9 wherein the adhesive is applied to a surface of the fibrous matrix in an amount ranging from about 0.0002 g of adhesive per cm² (about 0.001 gram of adhesive per square inch) of fibrous matrix to about 0.016 g of adhesive per cm² (about 0.1 gram of adhesive per square inch) of fibrous matrix.

11. The absorbent structure of one of claims 1 to 8 wherein the adhesive is nonwettable with the liquid to be contacted with the absorbent structure (6,58).

12. The absorbent structure of claim 11 wherein the adhesive is applied to a surface of the fibrous matrix in an amount ranging from about 0.0002 g of adhesive per cm² (about 0.001 gram of adhesive per square inch) of fibrous matrix to about 0.008 g of adhesive per cm² (about 0.05 gram of adhesive per square inch) of fibrous matrix.

13. The absorbent structure of one of the preceding claims wherein the absorbent structure exhibits an Absorbency Under Load value of at least about 15 grams of liquid per gram of the absorbent structure (6,58), preferably an Absorbency Under Load value of at least about 20 grams of liquid per gram of the absorbent structure.

14. The absorbent structure of one of the preceding claims wherein the absorbent structure exhibits a Capillary Tension Capacity value that is at least about 8 grams of liquid per gram of the absorbent structure, preferably a Capillary Tension Capacity value that is at least about 10 grams of liquid per gram of the absorbent structure.

15. The absorbent structure of one of the preceding claims wherein the liquid is water, synthetic urine, urine, menses, blood, or a 0.9 weight percent aqueous saline solution.

16. The absorbent structure of one of the preceding claims wherein the adhesive has an extractable fraction level that is less than about 0.1 weight percent of the total weight of adhesive.

17. The absorbent structure of one of the preceding claims wherein the hydrogel-forming polymeric material is selected from the group consisting of carboxymethyl cellulose and alkali metal salts of polyacrylic acid; wherein the fiber matrix comprises fibers selected from the group consisting of wood pulp, cellulose, cellulose acetate, cotton linter, nylon, rayon, and polyacrylonitrile fibers; wherein the adhesive is selected from the group consisting of thermoplastic hot-melt adhesives and reactive adhesives, is wettable with the liquid to be contacted with the absorbent structure, and is applied to a surface of the fibrous matrix in an amount ranging from about 0.0002 g of adhesive per cm² (about 0.001 gram of adhesive per square inch) of fibrous matrix to about 0.016 g of adhesive per cm² (about 0.1 gram of adhesive per square inch) of fibrous matrix; and wherein the liquid is water, synthetic urine, urine, menses, blood, or a 0.9 weight percent aqueous saline solution.

18. A process for preparing an absorbent structure comprising:
contacting a fibrous matrix, wherein the fibrous matrix is wettable with a liquid to be contacted with the absorbent structure, with an adhesive, wherein the adhesive is substantially non-soluble and is substantially non-dispersible in the liquid to be contacted with the absorbent structure; and
contacting the adhesive with a hydrogel-forming polymeric material, wherein the hydrogel-forming polymeric material becomes adhered to the fibrous matrix; wherein the absorbent structure exhibits an Absorbency Under Load value of at least about 12 grams of the liquid per gram of the absorbent structure and a Capillary Tension Capacity value that is at least about 6 grams of the liquid per gram of the absorbent structure.

19. The process of claim 18 further wherein the fibrous matrix comprises an outer surface and the adhesive is contacted with said outer surface of the fibrous matrix.

20. The process of claim 18 or 19 wherein the adhesive is wettable with a liquid to be contacted with the absorbent structure.

21. The process of claim 20 wherein the adhesive is applied to said surface of the fibrous matrix in an amount ranging from about 0.0002 g of adhesive per cm² (about 0.001 gram of adhesive per square inch) of fibrous matrix to about 0.016 g of adhesive per cm² (about 0.1 gram of adhesive per square inch) of fibrous matrix.

22. The process of claim 18 or 19 wherein the adhesive is nonwettable with a liquid to be contacted with the absorbent structure.

23. The process of claim 22 wherein the adhesive is applied to said surface of the fibrous matrix in an amount ranging from about 0.0002 g of adhesive per cm² (about 0.001 gram of adhesive per square inch) of fibrous matrix to about 0.008 g of adhesive per cm² (about 0.05 gram of adhesive per square inch) of fibrous matrix.

24. The process of one of the preceding claims wherein the hydrogel-forming polymeric material is selected from the group consisting of agar, pectin, and guar gum, carboxymethyl cellulose, alkali metal salts of polyacrylic acid, polyacrylamides, polyvinyl alcohol, ethylene maleic anhydride copolymers, polyvinyl ethers, hydroxypropyl cellulose, polyvinyl morpholinone, polymers and copolymers of vinyl sulfonic acid, polyacrylates, polyacrylamides, and polyvinyl pyrridines.

25. The process of one of the preceding claims wherein the absorbent structure comprises from about 5 to about 95 weight percent hydrogel-forming polymeric material, from about 5 to about 95 weight percent fibrous matrix, and from about 0.5 to about 30 weight percent adhesive, based on total weight of the hydrogel-forming polymeric material, fibrous matrix, and the adhesive in the absorbent structure.

26. The process of one of the preceding claims wherein the fiber matrix comprises fibers selected from the group consisting of wood pulp, cellulose, cellulose acetate, cotton linter, nylon, rayon, and polyacrylonitrile fibers.

27. The process of one of the preceding claims wherein the fibrous matrix is in the form selected from the group consisting of a batt of comminuted wood pulp fluff, a tissue layer, a hydroentangled pulp sheet, a mechanically softened pulp sheet, and a web structure.

28. The process of one of the preceding claims wherein the adhesive is selected from the group consisting of thermoplastic hot-melt adhesives and reactive adhesives.

29. The process of one of the preceding claims wherein the absorbent structure exhibits an Absorbency Under Load value of at least about 15 grams of liquid per gram of the absorbent structure.

30. The process of one of the preceding claims wherein the absorbent structure exhibits a Capillary Tension Capacity value that is at least about 8 grams of liquid per gram of the absorbent structure, preferably a Capillary Tension Capacity value that is at least about 10 grams of liquid per gram of the absorbent structure.

31. The process of one of the preceding claims wherein the liquid is water, synthetic urine, urine, menses, blood, or a 0.9 weight percent aqueous saline solution.

32. The process of one of the preceding claims wherein the adhesive has an extractable fraction level that is less than about 0.1 weight percent of the total weight of adhesive.

33. The process of one of the preceding claims wherein the hydrogel-forming polymeric material is selected from the group consisting of carboxymethyl cellulose and alkali metal salts of polyacrylic acid; wherein the fiber matrix comprises fibers selected from the group consisting of wood pulp, cellulose, cellulose acetate, cotton linter, nylon, rayon, and polyacrylonitrile fibers; wherein the adhesive is selected from the group consisting of thermoplastic hot-melt adhesives and reactive adhesives, is wettable, and is applied to a surface of the fibrous matrix in an amount ranging from about 0.0002 g of adhesive per cm² (about 0.001 gram of adhesive per square inch) of fibrous matrix to about 0.016 g of adhesive per cm² (about 0.1 gram of adhesive per square inch) of fibrous matrix; and wherein the liquid is water, synthetic urine, urine, menses, blood, or a 0.9 weight percent aqueous saline solution.
